# EUROPEAN PATENT APPLICATION

(11) **EP 4 124 622 A1**
(43) Date of publication of application: **01.02.2023**
(21) Application number: 21188498.6
(22) Date of filing: 29.07.2021
(51) Int. Cl.: C07K 16/18, C07K 16/28, G01N 33/532, C07K 16/30

(54) **RAPID HISTOLOGICAL DIAGNOSIS FOR ONCOLOGY THERAPY**

(71) Applicant: Fondazione IRCCS Istituto Nazionale dei Tumori, 20133 Milan (IT)
(72) Inventor: MILIONE, Massimo, 20133 MILANO (IT)
(74) Representative: Zaccaro, Elisabetta

(57) **Abstract**

The present invention concerns the field of oncology, and in particular tumor diagnosis for specific and tailored therapy.

In particular the invention describes a method for diagnosing a tumor in a subject, said method comprising the step of performing an in vitro immunohistochemical (IHC) analysis, wherein said IHC is carried out on a fresh frozen sample and with a specific set of antibodies.

The present invention further relates to a kit for IHC analysis comprising the specific panel of antibodies and instructions for use in the method of according to the invention.

In a further aspect, the invention relates to the use of a kit for providing a tumor diagnosis, wherein said tumor is chosen from the group consisting of breast, liver, testis, prostate, skin (melanoma), lung, thyroid, colon, colorectal, uterus, lymph node, bladder, pancreas, spleen, upper aerodigestive tract and stomach.

## Description

### FIELD OF THE INVENTION

The present invention concerns the field of oncology, and in particular tumor diagnosis for specific and tailored therapy.

In particular the invention describes a method for diagnosing a tumor in a subject starting by fresh tissue and not requiring the formalin fixed and paraffin embedded procedure (FFPE). In the method of the invention, fresh frozen samples are colored with the basic worldwide accepted method called Hematoxylin and Eosin (H&E) and in parallel tested by immunohistochemical (IHC) analysis. H&E and IHC are carried out on a fresh frozen sample and using a specific set of antibodies.

The present invention uses H&E as just performed during a routinary intraoperative examination and regarding IHC further relates to a kit for IHC analysis comprising the specific panel of antibodies and instructions for use in the method of according to the invention.

In a further aspect, the invention relates to the use of a kit for providing a tumor diagnosis, wherein said tumor is chosen from the group consisting of breast, liver, testis, prostate, skin (melanoma), lung, thyroid, colon, colorectal, uterus, lymph node, bladder, pancreas, spleen, upper aerodigestive tract and stomach.

### STATE OF THE ART

Despite constant advances in oncology, surgery and therapeutic approaches against tumors, the mortality rate remains high. A tailored therapeutic approach is requested. In more detail, the precise molecular map of each neoplasm deserves an adequate treatment. The worst clinical scenario is defined by late-stage oncological disease, associated with metastatic diffusion. In this situation proper therapeutic approach could be driven by the precise tumor phenotype (epithelial vs connective vs muscular vs nerve origin). For instance, an epithelial neoplasm is treated differently when the primary site is breast, lung or stomach. It is crucial to identify other specific variants (i.e., neuroendocrine, melanocytic or blood and lymphatic) that benefit from targeted drugs. Accuracy and promptness are equally important in case of transplant rejection. We can find the answer to the question above by analyzing a biopsy. Morphological information obtained from routine slides must be integrated with immunohistochemical (IHC) analysis. Our patients have to face the challenge of time: the fastest is the diagnosis, the more effective is the treatment.

Pathology Unit plays a key role, particularly in this situation, where a prompt diagnosis is crucial for the choice of the best therapeutic approach. A sample must be fixed in formalin and embedded in paraffin, in order to be morphologically evaluated, before immunohistochemical (IHC) staining can be performed. This requires at least a further day, considering exclusively technical times of execution in an ideal situation, in fact, standard Turn Around Time (TAT) for Regione Lombardia (Mod. Decreto di Regione Lombardia n. 1606/2019) ranges from 10 days for bioptic samples to more than 20 for histological ones. Overall, making a complex diagnosis can take up to 12-20 days. In cases when molecular diagnostic studies are required, up to 20 days may be necessary before the full set of information is available and treatment can be established.

The need and importance is increasingly required for accelerating the process to obtain a diagnosis, in order to reduce the time needed to set a therapeutic approach tailored to the patients' needs.

The object of the present invention is therefore the development of a method for rapid and definitive molecular diagnosis for the most personalized oncological treatment.

### SUMMARY OF THE INVENTION

The present invention concerns in a first aspect a method for diagnosing a tumor in a subject, said method comprising the step of performing an immunohistochemical (IHC) analysis, wherein said IHC is carried out:
- on fresh frozen samples,
- in a microfluidic staining device, and
- with a panel of antibodies.

The method of the present invention allows a Rapid Histological Diagnosis for Oncology Therapy, and has the acronym DIRTO from the Italian translation.

The method of the present invention surprisingly allows to:
- define whether the tumor is benign or malignant: using first the morphological examination on the H&E staining obtained from the first frozen section;
- identify the tumor type by using the best combination of IHC stainings with the aforesaid set of antibodies to provide the information required for the diagnosis. For example if the pathologist needs to distinguish a carcinoma from a mesothelioma (these tumours are both in the peritoneum and could be morphologically similar) the antibody to be used is claudine-4. A positive result in the neoplasm with claudine-4 tells us that we should consider the tumor a carcinoma, while with a negative result it can be considered a mesothelioma. If the pathologist has to distinguish between hepatocarcinoma and cholangiocarcinoma (both present in liver and often similar on H&E) he will use the Anti-Human Hepatocyte antibody and a positive result indicates hepatocarcinoma while a negative result is a cholangiocarcinoma);
- determine whether the tumor is in the organ or metastatic. In particular, if on a previous morphological analysis the pathologist suspected intestine origin or the tumor, he will use mainly the CDX2 antibody, if his suspect is breast he will use GATA 3 and so on.

In a second aspect, the present invention relates to a kit for IHC analysis comprising a panel of antibodies, such as: CDX2, Claudin-4, CK 7, CK 19, CK AE1/AE3, GATA-3, Anti-Human Hepatocyte, Anti-Human Ki67, p40, PAX 8, Synaptophysin and Anti-Thyroid Transcription Factor (TTF1), CD3, CD20, CD45, Estrogen Receptor, Progesteron Receptor and SOX10, said kit further comprising instructions for use in the method of according to the invention.

In a third aspect, the invention relates to the use of the present kit for providing a tumor diagnosis, wherein said tumor is chosen from the group consisting of breast, liver, testis, prostate, skin (melanoma), lung, thyroid, colon, colorectal, uterus, lymph node, bladder, pancreas, spleen, upper aerodigestive tract and stomach.

The problem underlying the present invention is that of making available an improved method for tumor diagnosis which allows to reduce time and provide a definitive molecular diagnosis for treating the patient in the most effective and efficient manner. This problem is solved by the present finding by the method and kit as identified in the attached claims.

### BRIEF DESCRIPTION OF THE DRAWINGS

The characteristics and advantages of the present invention will be apparent from the detailed description reported below, from the Examples given for illustrative and nonlimiting purposes, and from the annexed Figures 1-6, wherein:
Figure 1: shows IHC stainings for CK AE1/AE3, Claudin-4 and CDX2 on neoplastic colon, Ki-67 on healthy spleen and Synaptophysin on healthy pancreas - CDX2, Ki-67 and Synaptophysin showed a slightly fainter signal on LabSat^{™} IHC, but maintained their specificity.
Figure 2: shows IHC stainings for Hepatocytes, TTF1, p40, PAX8 on healthy liver, lung, skin and uterus, respectively, - TTF1 LabSat^{™} IHC showed a higher background reaction.
Figure 3: IHC stainings for CK7 and CK19 on healthy liver - LabSat^{™} IHC showed a higher background reaction but maintained their specificity for ductal structures.
Figure 4: IHC stainings for estrogen receptor, progesteron receptor and GATA3 on invasive breast cancer samples.
Figure 5: IHC stainings for CD3, CD20 and CD45 on healthy lymph node.
Figure 6: Comparison between the currently used workflow and the workflow of the method according to the DIRTO method of the present invention; the time indicated in the figure is only related to technical time of execution of the procedures.

### DETAILED DESCRIPTION OF THE INVENTION

In the metastatic setting several analyses might be necessary to identify the origin of the metastatic disease. Immunohistochemistry is the most useful tool to identify the expression of specific markers required for diagnosis. Using a microfluidic staining technology, we implemented a specific panel of cancer-specific biomarkers, to identify the primary tumor, with a Turn Around Time (TAT) in the range of 32 to 45 minutes, preferably of -35 minutes.

The present invention thus concerns an improved immunohistochemical staining method which allows to provide a rapid and definitive diagnosis. The method can be successfully carried out on a tissue sample from a surgical biopsy and result in a complete and definite diagnosis for the patient during the operation.

The present invention concerns in a first aspect a method for diagnosing a tumor in a subject, said method comprising the step of performing an in vitro immunohistochemical (IHC) analysis, wherein said IHC is carried out:
- on a fresh frozen sample,
- in a microfluidic staining device, and
- on a panel of antibodies.

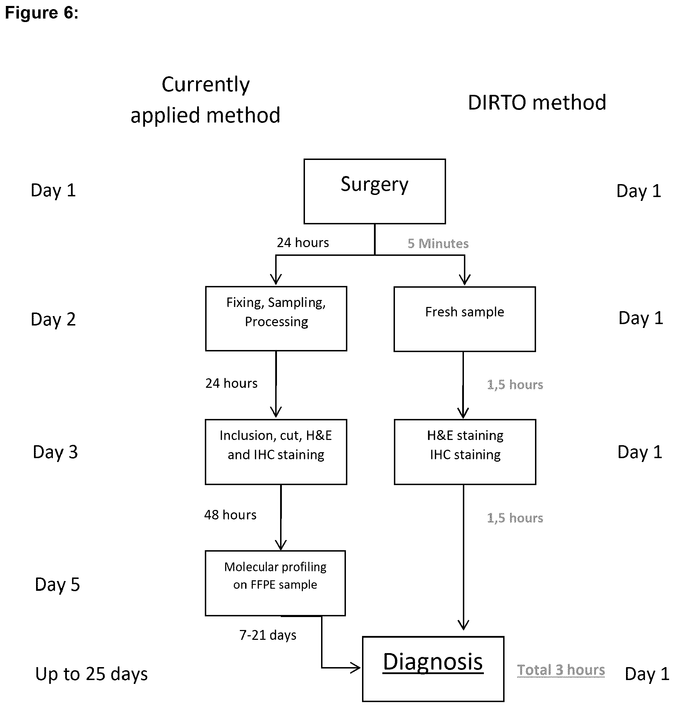

Morphological and immunohistochemical evaluations of biological material for the definition of the neoplastic phenotype, biopsies and surgical samples are of crucial importance for the pathological diagnosis and normally require 7 to 21 working days. The workflow which is currently used for diagnosis of surgical samples is schematically shown in Figure 6, which shows that with the DIRTO method of the invention the whole process takes only hours and allows to drastically reduce the time needed to make a diagnosis. The DIRTO method of the invention comfortably allows to comply with the requirements of the TAT indicated by Regione Lombardia (Mod. Decreto di Regione Lombardia n. 1606/2019).

The promptness of the aforementioned diagnosis guides the subsequent molecular and therapeutic approaches. The invention is thus directed to a more rapid, effective and complete method which drastically reduces the time required for the patient to receive a complete diagnosis, allowing him to receive a personalized therapy on the basis of the type of neoplasm from which he is affected.

In a preferred embodiment, in the method of the present invention the panel of antibodies consists of: CDX2, Claudin-4, CK 7, CK 19, CK AE1/AE3, GATA-3, Anti-Human Hepatocyte, Anti-Human Ki67, p40, PAX 8, Synaptophysin and Anti-Thyroid Transcription Factor (TTF1), CD3, CD20, CD45, Estrogen Receptor, Progesteron Receptor and SOX10 and said tumor is chosen from the group consisting of breast, liver, testis, prostate, skin (melanoma), lung, thyroid, colon, colorectal, uterus, lymph node, bladder, pancreas, spleen, upper aerodigestive tract and stomach.

In a further preferred embodiment, in the method of the present invention said panel of antibodies allows to determine the tumor phenotype, and said tumor phenotype is chosen from the group consisting of epithelial, connective, muscular or nervous origin.

The panel of antibodies used in the method according to the invention allows to determine the tumor morphological variant, such as epithelial, neuroendocrine, melanocytic, blood or lymphatic.

Wherein said method allows to identify the tumor type, whether it is a malignant or a benign tumor and whether it is primary or metastatic.

The result of the present finding is that by implementing the method of the present invention, said method allows the identification of a tailored oncological treatment for the patient, that can start the oncological treatment immediately after surgery.

In a preferred aspect the method of the present invention makes use of a LabSat^{®} Research, an ultra-rapid automated staining instrument for carrying out the method of the invention.

LabSat^{®} Research is an ultra-rapid automated staining instrument produced by Lunaphore Technologies, based on an innovative microfluidic technology that is capable of carrying out IHC/IF staining cycles within a few minutes, in a highly precise and reproducible manner.

Advantageously, the method according to the present invention, which can be carried out at the time of surgery with a Turn Around Time (TAT) of about 35 minutes, can provide the physicians with relevant information regarding the tumor phenotype and origin, allowing for specific decisions shortly after surgery. In addition the method allows the molecular analysis of the frozen tissue sample analized and therefore a tailored, fast and patient specific therapeutic approach. The method according to the present invention also allows to make a prognosis of the disease.

Therapeutic approaches of neoplastic diseases are extremely heterogeneous and clinicians need to have a correct and fast characterization of the stage of the disease and of the histotype of the tumor prior to commit to every kind of therapy. With the method of the present invention the clinician can allocate the most adequate pharmaceutical therapy and in-depth molecular analisys on the basis of the results of the immunohistochemistry.

The advantage of using the DIRTO method for subsequent molecular examination is that of not needing to use the formalin and paraffin step in order to have a diagnosis. Without the DIRTO method, the definition of the type of tumor and of the site is done on material previously fixed in formalin and then embedded in paraffin. This means that the subsequent molecular examinations will be performed starting from paraffinized material, requiring a longer, more complex extraction method for molecular examination and not always with an optimal yield. DIRTO, not needing formalin and paraffin passages to obtain the same results, allows starting from fresh material whose yield per molecular is optimal

In a second aspect, the present invention relates to a kit for IHC analysis comprising a panel of antibodies, wherein said panel of antibodies consist of: CDX2, Claudin-4, CK 7, CK 19, CK AE1/AE3, GATA-3, Anti-Human Hepatocyte, Anti-Human Ki67, p40, PAX 8, Synaptophysin and Anti-Thyroid Transcription Factor (TTF1), CD3, CD20, CD45, Estrogen Receptor, Progesteron Receptor and SOX10. said kit further comprising instructions for use in the method of according to the invention.

In a third aspect, the invention relates to the use of the present kit for providing a tumor diagnosis, wherein said tumor is chosen from the group consisting of breast, liver, testis, prostate, skin (melanoma), lung, thyroid, colon, colorectal, uterus, lymph node, bladder, pancreas, spleen, upper aerodigestive tract and stomach.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention.

### Example 1. Rationale for the selection of antibodies

According to the Italian Department of Health, breast cancer, colorectal cancer, lung cancer, gynecological cancer (endometrial) and melanocytic cancer were the 5 tumors most frequently diagnosed in 2019 (Table 1): this figure is similar to the epidemiological distribution of the same tumors worldwide and it has been unchanged for years. With this premise, we decided to implement the panel of antibodies we can use with LabSat^{™} Research, focusing at first on breast, colorectal, lung, endometrial and skin (melanocytic or squamous) neoplasms.

**Table 1 - Adapted and translated from http://www.salute.gov.it/portale/home.html**

| Rank | Male | | | Female | | |
|---|---|---|---|---|---|---|
| | Age | | | Age | | |
| | 0-49 | 50-69 | 70+ | 0-49 | 50-69 | 70+ |
| **Total cases** | **100% n=13.297** | **100% n=80.905** | **100% n=111.565** | **100% n=22.430** | **100% n=64.236** | **100% n=79.815** |
| 1° | Testis 12% | Prostate 22% | Prostate 19% | Breast 40% | Breast 35% | Breast 22% |
| 2° | Skin (Melanoma) 9% | Lung 14% | Lung 17% | Thyroid 16% | Colorectal 11% | Colorectal 16% |
| 3° | Thyroid 8% | Colorectal 12% | Colorectal 14% | Skin (melanoma) 7% | Uterus (body) 7% | Lung 7% |
| 4° | LNH 8% | Bladder 11% | Bladder 12% | Colorectal 4% | Lung 7% | Pancreas 6% |
| 5° | Colorectal 7% | Upper aerodigestive tract 5% | Stomach 5% | Uterus (cervix) 4% | Thyroid 5% | Stomach 5% |

### Primary antibodies and tissue selection

To assess the efficacy of DIRTO, we selected test tissues with a well know antigenic profile. Both healthy and neoplastic samples were selected; neoplastic tissues were previously evaluated by a pathologist and specimens were collected when in excess to pathologic diagnosis. To properly evaluate the specificity of the method, both surgical samples and samples from small biopsies were selected (Table 2).

**Table 2 - Tissues selected for the analysis.**

| **Antibodies** | **Samples** |
|---|---|
| TTF1 | Healthy lung (s) |
| CK 7 | Healthy liver (b) |
| CK 19 | Healthy liver (b) |
| CK AE1/AE3 | Neoplastic colon (s) |
| Claudin-4 | Neoplastic colon (s) |
| Anti-Human Hepatocyte | Healthy liver (b) |
| GATA-3 | Breast cancer (s) |
| Anti-Human Ki67 | Healthy spleen (s) |
| p40 | Healthy skin (s) |
| PAX8 | Healthy uterus (s) |
| CDX2 | Neoplastic colon (s) |
| Synaptophysin | Healthy pancreas (s) |
| CD3 | Healthy lymph node (s) |
| CD20 | Healthy lymph node (s) |
| CD4 | Healthy lymph node (s) |
| Estrogen Receptor | Breast cancer (s) |
| Progesteron Receptor | Breast cancer (s) |
| SOX10 | Melanoma (s) |
| b: biopsy; S: surgical sample | |

The primary antibodies used in the study are the following: CDX2, Claudin-4, CK 7, CK 19, CK AE1/AE3, GATA-3, Anti-Human Hepatocyte, Anti-Human Ki67, p40, PAX 8, Synaptophysin, Anti-Thyroid Transcription Factor (TTF1), CD3, CD20, CD45, Estrogen Receptor and Progesteron Receptor, CD3, CD20, CD45, Estrogen Receptor, Progesteron Receptor and SOX10 (Table 3).

**Table 3 - Antibodies dilution for LabSat^{™} Research and routine IHC staining**

| **Antigens** | **Code Number** | **Clone** | **Source** |
|---|---|---|---|
| Anti-Thyroid Trascription Factor (TTF1)(M) | M3575 | 8G7G3/1 | Dako,Agilent, Denmark |
| Cytokeratin 7 (M) | M7018 | OV-TL 12/30 | Dako,Agilent, Denmark |
| Cytokeratin 19 (M) | M0888 | RCK108 | Dako,Agilent, Denmark |
| Cytokeratin (M) | M3515 | AE1/AE3 | Dako,Agilent, Denmark |
| Claudin 4 (M) | 32-9400 | 3E2C1 | Invitrogen |
| Hepatocyte (M) | M7158 | OCH1E5 | Dako,Agilent, Denmark |
| GATA3 (M) | CM 405 A,B | L50-823 | Biocare Medical |
| Ki67 Antigen (M) | M7240 | Mib-1 | Dako,Agilent, Denmark |
| P40 (M) | ACI 3066 A,C | BC28 | Biocare Medical |
| PAX8 (M) | 60145-4-Ig | Ag0306 | Proteintech |
| CDX2 (M) | M3636 | DAK-CDX2 | Dako,Agilent, Denmark |
| Synaptophisin (M) | M7315 | Dak-Synap | Dako,Agilent, Denmark |
| CD3 (P) | A0452 | Polyclonal | Dako, Agilent, Denmark |
| CD20 (M) | M0755 | L26 | Dako,Agilent, Denmark |
| CD45 (M) | GA751 | 2B11+PD7/26 | Dako,Agilent, Denmark |
| Estrogen Receptor (M) | M3643 | EP1 | Dako,Agilent, Denmark |
| Progesteron Receptor (M) | M3568 | PGR1294 | Dako,Agilent, Denmark |
| SOX10 (M) | AP10963C | BC34 | GenNova |
| M: Monoclonal P: Policlonal | | | |

### Example 2. Sample preparation

Fresh human tissue samples of lung, liver, colon, breast, spleen, skin, uterus, pancreas and lymph node were obtained from surgical procedures at our institution. Tissue specimens were selected and sampled by a pathologist and frozen with PrestoCHILL (Milestone Medical). Samples were embedded in an histo-cassette and sectioned with a cryostat to obtain 4-5 µm-thick sections mounted on commercially available charged slide (Thermo Scientific Superfrost Plus).

One section was then stained with fast H&E staining and evaluated by a pathologist to assess the adequacy of the sample.

Sections for DIRTO IHC were fixed for 3 min in 10% neutral buffered formalin and then washed with water for 1 min. All tissue samples were then fixed in neutral buffered formalin, processed as routine samples and embedded in paraffin (FFPE) for the standard IHC staining.

### Example 3. Immunohistochemistry analysis

IHC stainings on fresh frozen samples were made with the LabSat^{™} Research device (Lunaphore Technologies SA) and confirmed on FFPE slides with Dako Autostainer Link 48 (Dako, Agilent). The LabSat^{™} Research device IHC was performed using commercially available antibodies and peroxidase block, protein block, hematoxylin and Mouse secondary antibodies from EnVision^{™} FLEX+ (Dako, Agilent) kit; Rabbit secondary antibody. Immunoreactions were visualized using DAB and counterstained with hematoxylin. Unlike the suggested LabSat^{™} Research protocol for frozen section we added an antigen retrieval step with Lunaphore's Antigen Retrieval pH9 10x for FFPE samples (changing the dilution from 1:10 to 1:100), for 2 to 8 minutes, depending of the antibody, expressly validated for each IHC protocol. Moreover, rabbit conjugated antibodies (Estrogen and PAX8) were validated, using EnVision FLEX+ Rabbit Linker DAKO for 4 minutes.

Regarding standard IHC, antigen unmasking was made with Dako PT-link, EnVision^{™} FLEX Target Retrieval Solution with High or Low pH solutions, with variable temperatures and time, depending on the staining. Immunoreactions were then visualized with EnVision^{™} FLEX+ (Dako, Agilent). All the slides were counterstained with hematoxylin.

DIRTO were performed within 35 minutes, while standard DAKO IHC needed a Turn Around Time (TAT) of 21-22 hours, depending of antigen retrieval and primary antibody incubation time. The selected antibodies were tested at different concentrations and a blind test by two different pathologists (LC, MM) with experience in IHC quality controls was performed to determine optimal concentrations.

### Results

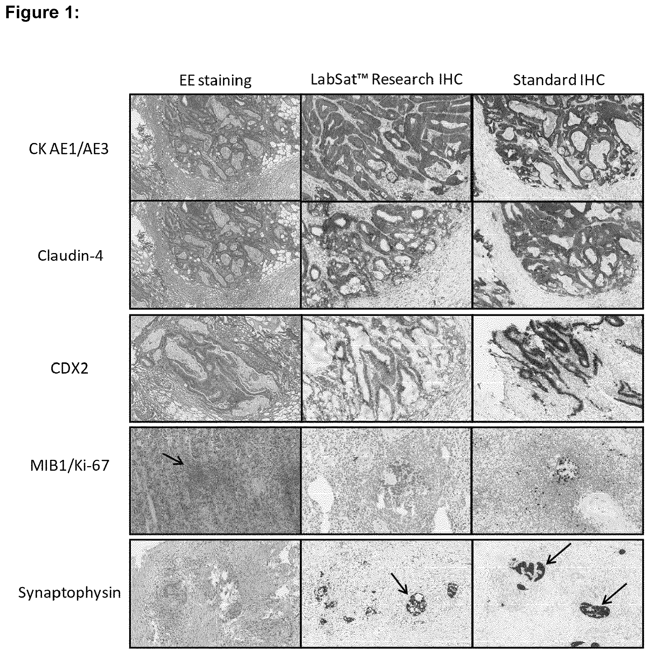

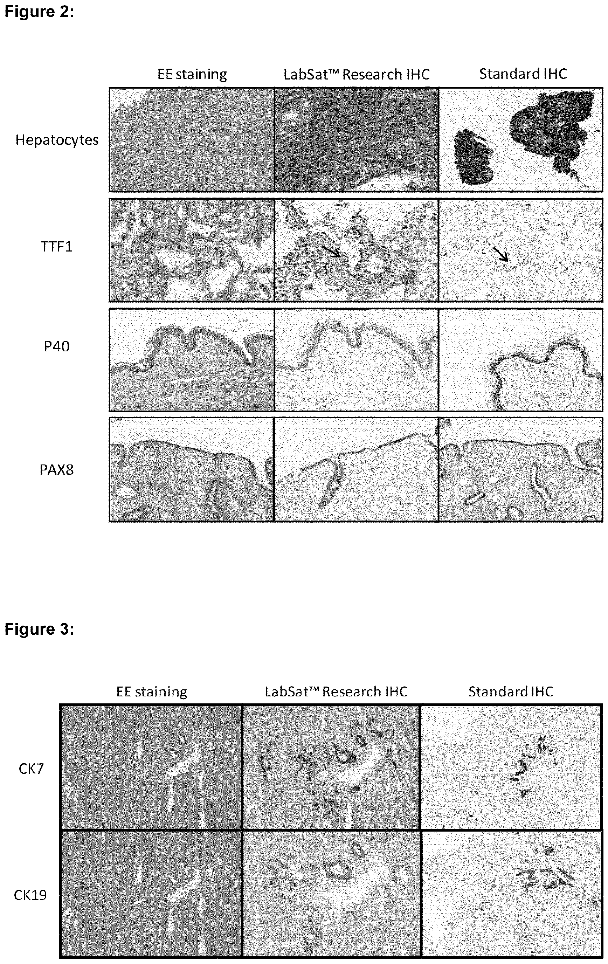

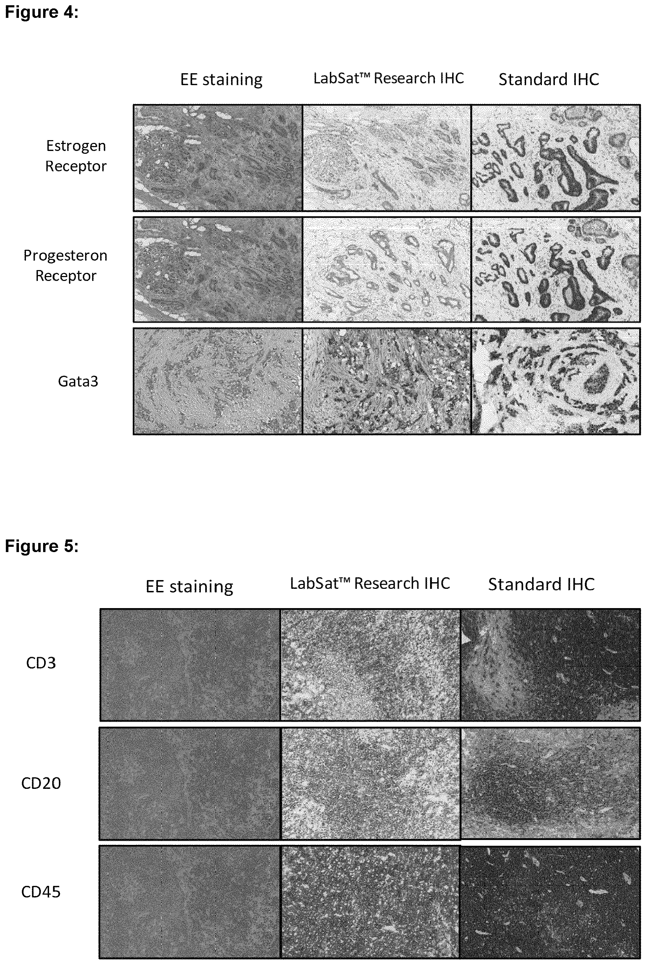

DIRTO has 18 stainings available (Table 3) enabling the possibility of a diagnostic use for the evaluation of metastasis origin and histotype covering both mouse conjugated antibodies with cytoplasmic and nuclear expression (CDX2, GATA3, Ki-67, p40, PAX8, TTF1, Estrogen and Progesteron Receptor) antibodies with rabbit as secondary antibody (Estrogen Receptor and PAX8). All the IHC on fresh frozen samples showed an expression comparable to standard IHC. Standard IHC protocols require specific antigen retrieval conditions, with different time and temperature of retrieval, buffers and antibody incubation time. To obtain optimal results each DIRTO antibody protocol was tested at various concentrations and with the addition of an high pH antigen retrieval step (not present in the suggested frozen IHC protocol), made with the standard antigen retrieval solution provided by Lunaphore Research for FFPE IHC with a higher dilution. Our protocol for DIRTO IHC was standardized for all the antibodies, maintaining a high reproducibility and keeping the staining adequate for a proper diagnosis. All the protocols were validated on positive samples with known antigenic expression and tested at various concentrations until the staining was comparable to standard FFPE IHC. Both pathologists evaluated in blind all the stainings and selected the protocols with the result comparable to standard IHC for specificity and intensity (Figures 1, 2, 3, 4, 5). All the antibodies validated to date were selected for their well-known utility for pathological diagnosis, but can be further implemented with virtually every mouse- and rabbit-conjugated antibody. After validation of the protocols, in agreement with surgeons and to meet clinical requests, DIRTO IHC has been used on clinical bioptic samples, mostly obtained by endoscopic ultrasound-fine needle aspiration (EUS-FNA) or endobronchial ultrasound guided needle biopsy (EBUS-TBNA), from different anatomical structures including lymph nodes, liver, intestine, lung and soft tissues. DIRTO were made only when at least two bioptic samples with adequate dimensions were available for each patients and confirmed on the definitive FFPE sample. Moreover, IHC stainings were made with appropriate positive controls to assess the adequacy of the procedure. All the samples were adequate for formalin fixation and paraffin embedding after the fast frozen IHC protocol, and standard IHC confirmed the initial evaluation in all cases for both positive and negative results.

From the above description and the above-noted examples, the advantage attained by the product described and obtained according to the present invention are apparent.

The present invention therefore resolves the above-lamented problem with reference to the mentioned prior art, offering at the same time numerous other advantages, including making possible the development of a diagnostic method capable of predicting the therapeutic response so to refine not only the diagnostics but above all direct the best therapeutic choice.

## Claims

1. A method for diagnosing a tumor in a subject, said method comprising the step of performing an in vitro immunohistochemical (IHC) analysis, wherein said IHC is carried out:
- on a fresh frozen sample,
- in a microfluidic staining device, and
- on a panel of antibodies.

2. The method according to claim 1, wherein said panel of antibodies consists of: CDX2, Claudin-4, CK 7, CK 19, CK AE1/AE3, GATA-3, Anti-Human Hepatocyte, Anti-Human Ki67, p40, PAX 8, Synaptophysin and Anti-Thyroid Transcription Factor (TTF1), CD20, CD45, Estrogen Receptor, Progesteron Receptor and SOX10.

3. The method according to any one of claims 1 or 2, wherein said tumor is chosen from the group consisting of breast, liver, testis, prostate, skin (melanoma), lung, thyroid, colon, colorectal, uterus, lymph node, bladder, pancreas, spleen, upper aerodigestive tract and stomach.

4. The method according to claim 3, wherein said panel of antibodies allows to determine the tumor phenotype.

5. The method according to any one of claims 1 or 2, wherein said tumor phenotype is chosen from the group consisting of epithelial, connective, muscular or nervous origin.

6. The method according to claim 5, wherein said panel of antibodies allows to determine the tumor morphological variant.

7. The method according to any one of claims 1-6, wherein said tumor morphological variant is chosen from the group consisting of epithelial, neuroendocrine, melanocytic, blood or lymphatic.

8. The method according to any one of claims 1-7, wherein said method allows the identification of a tailored oncological treatment.

9. A kit for IHC analysis comprising a panel of antibodies, wherein said panel of antibodies consist of: CDX2, Claudin-4, CK 7, CK 19, CK AE1/AE3, GATA-3, Anti-Human Hepatocyte, Anti-Human Ki67, p40, PAX 8, Synaptophysin and Anti-Thyroid Transcription Factor (TTF1), CD20, CD45, Estrogen Receptor, Progesteron Receptor and SOX10, said kit further comprising instructions for use in the method of claims 1 to 8.

10. Use of the kit according to claim 9 for providing a tumor diagnosis, wherein said tumor is chosen from the group consisting of breast, liver, testis, prostate, skin (melanoma), lung, thyroid, colon, colorectal, uterus, lymph node, bladder, pancreas, spleen, upper aerodigestive tract and stomach.
